# EUROPEAN PATENT SPECIFICATION

(11) **EP 3 795 144 B1**
(45) Date of publication and mention of the grant of the patent: **22.01.2025**
(21) Application number: 20020338.8
(22) Date of filing: 24.07.2020
(51) Int. Cl.: A61K 9/70, A61K 47/06, A61K 47/44, A61P 27/16

(54) **EAR SPRAY DEVICE AND EAR PROTECTION COMPOSITION FOR PREVENTING WATER RELATED EAR CANAL DISORDERS**
OHRSPRÜHVORRICHTUNG UND GEHÖRSCHUTZZUSAMMENSETZUNG ZUR VERHINDERUNG WASSERBEDINGTER GEHÖRGANGSSTÖRUNGEN
DISPOSITIF DE PULVÉRISATION D'OREILLE ET COMPOSITION DE PROTECTION DE L'OREILLE POUR PRÉVENIR DES TROUBLES DU CANAL AUDITIF LIÉS À L'EAU

(30) Priority: 09.09.2019 US 201916564251
(43) Date of publication of application: 24.03.2021
(73) Proprietor: LiquidPro Ltd, Valletta VLT1455 (MT)
(72) Inventor: Maier, Tradian Almasanu, 80639 Munich (DE)
(74) Representative: Pintz, György

(56) References cited:
- EP-B1- 1 778 315
- WO-A1-2013/065051
- WO-A1-97/01348

## Description

### FIELD OF THE INVENTION

The present invention pertains to prevention for otic disorders, and more specifically to a composition for preventing water related ear canal disorders. Furthermore, the present invention relates to an ear disorder prevention composition dispensing device.

### DESCRIPTION OF THE RELATED ART

Presence of the cerumen glands in the human ear canal is important to maintain the overall health of the ear canal and thus protects the ear from various common disorders. The cerumen glands secretes a waxy material known as cerumen or ear wax which acts as a trapping agent for various outside agents such as airborne debris. Further, the cerumen also acts as a water repellent and also acidifies the epithelial surface of the canal, thus reducing the chances of bacterial and fungal infections within the ear canal.

Further, the fine hair present within the outer ear canal impels the ear wax gradually outwardly, and this process also helps in removing any dirt particles that has been trapped inside the ear canal. Finally, the ear wax is expelled out of the outer ear canal, which slowly dries and thus falls out of the ear. This whole process removes the foreign particles from the ear canal and naturally cleans the ear.

Commonly Otitis externa is referred to as "swimmer's ear", which occurs due to repeated exposure of ear canal to water during swimming and related water activities. The condition makes the ear canal more vulnerable to inflammation and leads to disorders caused by bacteria and fungi. Moreover, during humid conditions, the water trapped in the ear canal also contracts a swimmer's ear, and further worsens the inflammation and disorders.

It is always difficult to remove the trapped water as it stays in the ear and defies gravity by refusing to flow outside of the ear. Thus, the trapped water in the ear provides ambient condition (moist and warm environment) which is perfect for bacteria to grow. As a result, bacteria and fungi use water as the primary vehicle for the beginning of bacterial and fungus growth resulting in the Swimmer's Ear disorders (Otitis Externae).

It is found that the particular bacteria causing otitis externa are *Pseudomonas Aeruginosa* and *Staphylococcus Aureus,* and other gram-positive and gram-negative species of bacteria. Moreover, *Candida Albicans* and *Aspergillus* species are the most common fungal pathogens responsible for the ear disorder.

Otitis externa causes inflammation (redness and swelling) of the external ear canal. Several other symptoms relating to this condition are ear pain, itching and irritation in and around the ear canal, redness and swelling of the outer ear and ear canal, a feeling of pressure and fullness inside the ear, scaly skin in and around the ear canal, which may peel off, discharge from the ear, which can be either thin and watery or thick and pus-like, tenderness when the ear or jaw is moved, swollen and sore glands in the throat, hearing loss and the like symptoms.

Another ear related condition is "Surfer's ear", which is the common name for an exostosis or abnormal bone growth within the ear canal.

In earlier times, the solution to the problem of the above conditions has been approached by various methods directed at removal of water after swimming. Other approaches are directed towards medicinal treatment after the condition has occurred.

Typical medicinal treatments of prior art involve myriad over-the-counter and prescription-only pharmaceutical agents available for symptomatic relief of the symptoms of otitis externa and surfer's ear. Usually, the treatment involves prescribing either antibiotic or antifungal ear drops or other forms of ear drops and therapies which either contain active medicinal ingredients and/or preservatives which in many cases can cause further allergies or sensitivities to some users.

However, regular use of antibiotic or antifungal compounds is not considered safe for the human body. Especially, when the person is regularly going for water related activities such person can't avoid the threat of continuous and longtime use of antibiotic or antifungal compounds.

Moreover, sometimes treatment of surfer's ear becomes far more complex and often involves surgery under anesthesia to remove the obstructing ear canal bone by the use of a binocular microscope. Most ear surgeons use a drill to remove the bone. Depending on the condition of the ear canal and the surgical technique used, the ear canal may require several weeks to several months to heal. Unprotected exposure of ear canals to cold water and wind after treatment can lead to regrowth of bone and the need for repeated operations on the same ear.

Options available until now to help prevent water penetration into the ear involve use of ear plugs when swimming or wear a swimming cap to cover ears and protect them from water ingress.

Other prevention methods are to use waterproofing (water repellent and water drying), ear sprays/drops applied before or after exposure to water creating waterproof barriers or post-water exposure drying in the ear canal which facilitates the flow of water out of the ear. These barriers prevent ear discomfort and infections by reducing exposure to trapped water. Further, other prevention method includes use of hairdryer to dry the ear after the trapping of water inside the ear canal.

There are other methods to prevent water penetration into the ear, such as disclosed in European patent no. EP1778315B1**,** which provides a dispensing device for dispensing a pre-defined quantity of oil. The oil comprises a mineral oil and vegetable oil, which is introduced into ears to avoid water entrapment. These devices prevent water borne bacterial ear infections as well as protect the ears from dirty pool water, reducing itchy and irritable ears and hearing loss after swimming.

Further, US patent no. US4278664 focuses on preventative compositions for preventing Otitis externa by eliminating discomfort of water remaining in the external canal, by applying a liquid to the skin surface of the external auditory canal of a user's ear.

It is evident there are a number of formulations which act as a barrier to reduce water entrapment in the ears and reduce infection and discomfort.

As discussed above there are several water barrier systems and compositions to prevent the onset of ear conditions such as otitis externa, surfer's ear, other common ear canal disorders and infections. Therefore, it is important to note the effectiveness, side effects and risk of these known systems and compositions.

Most of these systems and compositions as known do not ensure complete ear protection as well as are cumbersome for some users to use due to specific shape of their ear, and/or specific skin type, and/or user's specific requirements.

Therefore, there is a need for developing ear protection compositions and systems which are cosmeceutical in nature, and free from side effects associated with pharmaceutical compositions.

Although there are several water repelling ear protection systems and compositions available in the market, still there is a need to develop an easy to use and effective system and composition for protecting the ear from such water related problems.

There is also a need of a system and composition which can be used by every user despite of their age group, their ear shape, and/or ear skin type. There is also a need of a system and/or composition which can be applied by the user himself/herself before entering into water during swimming or surfing activities. Accordingly, the system and composition as disclosed in the present invention confer the benefits which outweigh the previously identified systems and compositions.

### SUMMARY

The following presents a simplified summary of the claimed subject matter in order to provide a basic understanding of some aspects of the claimed subject matter. This summary is not an extensive overview of the claimed subject matter. It is intended to neither identify key or critical elements of the claimed subject matter nor delineate the scope of the claimed subject matter. Its sole purpose is to present some concepts of the claimed subject matter in a simplified form as a prelude to the more detailed description that is presented later.

In view of the aforesaid needs and shortcoming of the state of the art in an aspect, the present invention provides a composition and a device to prevent the problem of trapping of water in the ear canal of a person and preventing ear from water borne infections.

Various embodiments of the present invention disclose a composition for use in preventing water related ear canal disorders, a preventing ear spray comprising said composition for the defined use, and a spray device for dispensing a pre-defined quantity of said composition for the defined use. The scope of the present invention is limited by the appended claims.

In accordance with the present invention, the ear protection composition includes a mineral oil within the range of 98.0 percent w/v to 99.9 percent w/v, and a vegetable oil within the range of 2.0 percent w/v to 0.1 percent w/v. Preferably, the mineral oil is 99.9 percent w/v and the vegetable oil is 0.1 percent w/v,

The mineral oil is *Paraffinum Liquidum* and the vegetable oil is an *Oreganum Oil.* Moreover, the *Oreganum Oil* is an essential oil selected from at least one of the species of the *Oreganum* family selected from *Oreganum Compactum, Oreganum Vulgare,* and a combination thereof.

The ear protection composition forms a thin water repellent layer topically to an external ear canal of the user. The *Oreganum Oil* is an antimicrobial agent, a natural antibacterial agent, an antifungal agent and an antiseptic agent effective against a plurality of bacteria, a plurality of fungi, a plurality of viruses and alike microbes responsible for disorders of human ear canal.

In the present invention, the ear protection composition is for use in the prevention of water-related ear canal disorders, wherein the said composition is applied in an ear canal before a user enters into water.

In accordance with another embodiment of the present invention, an ear spray is also disclosed for protecting the ear from water entrapment. The said ear spray comprises a water repellent agent, and an antimicrobial agent, wherein the said ear spray is applied in the ear canal by a metered dosage of 0.1 ml to 1.0 ml before the user enters in the water. The said ear spray is characterized in that the water repellent agent is *Paraffinum Liquidum* in the range of 98.0 percent w/v to 99.9 percent w/v, and the antimicrobial agent is an *Oreganum Oil in* the range of 2.0 percent w/v to 0.1 percent w/v.

In accordance with yet another embodiment of the present invention, the present invention further discloses an ear spray device dispensing a pre-defined quantity of the ear protection composition in the ear canal of the user.

The said ear protection device includes a reservoir containing the said ear protection composition, a spray nozzle and a pump mechanism. The ear protection composition comprises a water repellent agent, and an antimicrobial agent.

The water repellent agent is *Paraffinum Liquidum* within the range of **99.6** percent w/v to 99.9 percent w/v, and the antimicrobial agent is an *Oreganum Oil* within the range of 0.4 percent w/v to 0.1 percent w/v. Preferably, the *Paraffinum Liquidum* 99.9 percent w/v and the *Oreganum Oil* is 0.1 percent w/v.

The spray nozzle of the present invention is operatively coupled to the reservoir, and adapted to spray the ear protection composition inside the ear canal of the user when the said ear spray device is in either a substantially upright or a substantially inverted position. Furthermore, the device includes a pump mechanism connecting the reservoir with the spray nozzle, and is adapted to draw the ear protection composition from the reservoir and spray the said composition in metered dosage via the spray nozzle inside the ear canal of the user. In a specific embodiment, the ear protection composition includes 99.9 percent w/v of *Paraffinum Liquidum* and 0.1 percent w/v of an *Oreganum Oil,* forming a thin water repellent layer inside the ear canal of the user.

In accordance with one or more embodiments of the present invention, the pre-defined quantity of said ear protection composition applied topically inside the ear canal of the user has a metered dosage of 0.1 ml to 1.0 ml.

The ear spray device is easily used by the user itself to apply a metered dosage of the ear spray before entering into the water.

### DESCRIPTION OF THE DRAWING

So that the manner in which the above recited features of the present invention is to be understood in detail, a more particular description of the invention, briefly summarized above, may be had by reference to embodiments, some of which are illustrated in the appended drawings. It is to be noted, however, that the appended drawings illustrate only typical embodiments of this invention and are therefore not to be considered limiting of its scope. The invention may admit other equally effective embodiments as long as they fall within the scope of the appended claims.

A better understanding of the present invention can be obtained when the following detailed description of the preferred embodiments is considered in conjunction with the following drawings. Moreover, the advantages and features of the present invention will become better understood with reference to the following detailed description taken in conjunction with the accompanying drawings, in which:
Figure 1 illustrates a sectional view of the ear spray device in accordance with one or more embodiments of the present invention.
Figure 2 illustrates a third angle projection of the ear spray device in accordance with one or more embodiments of the present invention.
Figure 3 illustrates a perspective view of the ear spray device in accordance with one or more embodiments of the present invention.

### DESCRIPTION OF THE INVENTION

The following detailed description is, therefore, not to be taken in a limiting sense, and the scope of the present disclosure is defined by the appended claims.

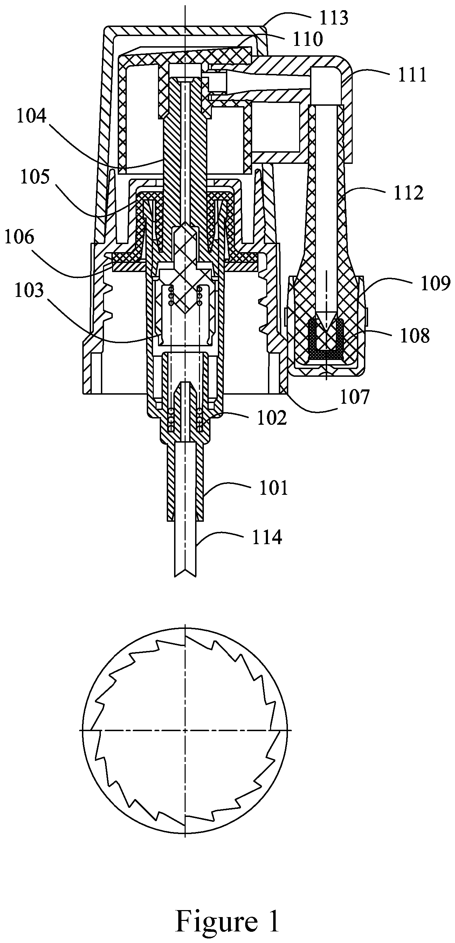

Various embodiments of the present invention relate to a composition for use in preventing water-related ear canal disorders, a preventing ear spray comprising said composition for the defined use, and a spray device for dispensing a pre-defined quantity of said composition for the defined use. The scope of these embodiments is determined by the appended claims. Moreover, the principles of the present invention and their advantages are best understood by referring to Figure 1.

In the following detailed description of illustrative or exemplary embodiments of the disclosure, specific embodiments in which the disclosure may be practiced are described in sufficient detail to enable those skilled in the art to practice the disclosed embodiments. For example, specific details such as specific method steps, structures, compositions, elements, and connections are presented herein. However, it is to be understood that the specific details presented need not be utilized to practice the embodiments of the present disclosure. The scope of the present invention is limited by the appended claims. Embodiments not covered by the claims are not according to the invention.

The invention will now be described with respect to various embodiments. The following description provides specific details for a thorough understanding of, and enabling description for, these embodiments of the invention. However, one skilled in the art will understand that the invention may be practiced without these details. In other instances, well-known structures and functions are not shown or described in detail to avoid unnecessarily obscuring the description of the embodiments of the invention.

It is intended that the terminology used in the description presented be interpreted in its broadest reasonable manner, even though it is being used in conjunction with a detailed description of certain specific embodiments of the invention. Certain terms may even be emphasized below; however, any terminology intended to be interpreted in any restricted manner will be overtly and specifically defined as such in this Detailed Description section.

The terms "a" and "an" herein do not denote a limitation of quantity, but rather denote the presence of at least one of the referenced item.

The terms "having", "comprising", "including", and variations thereof signify the presence of a component.

References within the specification to "one embodiment," "an embodiment," "embodiments," or "one or more embodiments" are intended to indicate that a particular feature, composition, structure, or characteristic described in connection with the embodiment is included in at least one embodiment of the present disclosure. The appearance of such phrases in various places within the specification are not necessarily all referring to the same embodiment, nor are separate or alternative embodiments mutually exclusive of other embodiments. Further, various features are described which may be exhibited by some embodiments and not by others. Similarly, various requirements are described which may be requirements for some embodiments but not for other embodiments. Embodiments not falling within the claimed subject-matter are not part of the invention.

The present invention discloses a composition for use in the prevention of water-related ear canal disorders, a preventing ear spray comprising said composition for the defined use, and a spray device for dispensing a pre-defined quantity of said composition for the defined use, according to the claims.

Figure 1 illustrates a sectional view of the ear spray device having the ear protection composition for preventing waterborne ear canal disorders in accordance with one or more embodiments of the present invention.

Particularly, the device is an ear spray device useful in preventing common ear canal disorders caused due to trapping of water in the ear canal of the user.

In accordance with one or more embodiments of the present invention, the ear protection composition includes 99.6 percent w/v to 99.9 percent w/v of *Paraffinum Liquidum* and 0.4 percent w/v to 0.1 percent w/v of an *Oreganum Oil.*

Preferably, the *Paraffinum Liquidum* 99.9 percent w/v and the *Oreganum Oil* 0.1 percent w/v.

The said liquid paraffin used in the said composition is an oily, transparent and colorless liquid obtained in the process of petroleum distillation. At room temperature it is odorless and tasteless although if heated it will exert a petroleum smell. Due to its chemical properties, it has many uses including industrial, medical as well as cosmetics.

Furthermore, due to its oily (emollient) properties and water resistance, the said liquid paraffin can coat skin surfaces and membranes to naturally repel and dissipate water, which makes it an essential ingredient in the composition to form a waterproof barrier in the ear canal. The other advantage is that it is not readily absorbed through the skin or mucous membranes into the body and is non-toxic.

Moreover, the said *Oreganum Oil* is an essential oil selected from at least one of the species of the *Oreganum* family selected from an *Oreganum Compactum,* an *Oreganum Vulgare,* or combination thereof.

The said oregano oil is usually derived from the leaves and flowers of Oregano (*Origanum Vulgare*)*,* a hardy perennial herb, and a member of the mint (Lamiaceae) family. The oil contains compounds called phenols, terpenes and terpenoids which are known to have powerful antioxidant properties and are responsible for its distinctive fragrance. Some of the key components of the said oil are as follows: -
- Carvacrol - this is the most abundant phenol in oregano oil, and it has been shown to inhibit the growth of several types of bacteria.
- Thymol - this is known to be a natural antifungal compound and can also support the immune system and protect against toxins.
- Rosmarinic acid - this is known to be a powerful antioxidant that helps protect against damage caused by free radicals.

The said composition of the present invention forms a thin water repellent layer topically to an external ear canal of the user. Also, *Oreganum Oil* is an antimicrobial agent, a natural antibacterial agent, an antifungal agent and an antiseptic agent effective against a plurality of bacteria, a plurality of fungi, a plurality of viruses and microbes responsible for problems of said ear canal.

In accordance with another embodiment of the present invention, the ear protection composition **is** applied in a metered dose in the ear canal before entering into water. The said metered dosage of the ear protection composition is of at least 0.1 ml to1.0ml.

Particularly, as described above, the composition has a combination of oils comprising a water repellent agent and an antimicrobial agent. The water repellent agent is *Paraffinum Liquidum* in the range of 98.0 percent w/v to 99.9 percent w/v, and the antimicrobial agent is an *Oreganum Oil* in the range of 2.0 percent w/v to 0.1 percent w/v. Subsequently, the ear protection composition forms a thin water repellent layer topically to an external ear canal of a user.

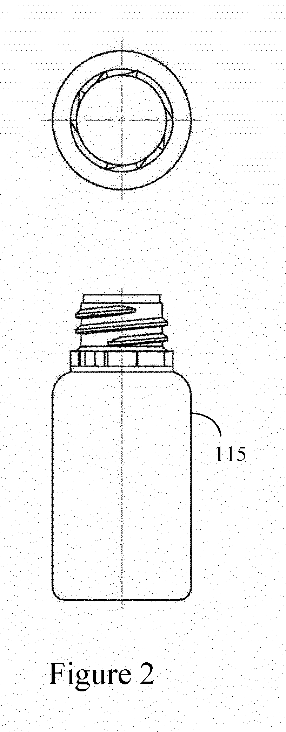

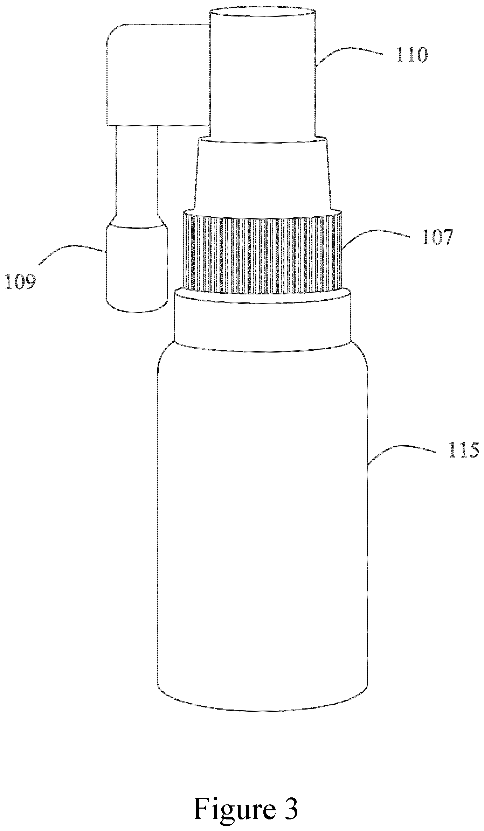

In accordance with yet another embodiment of the present invention, the present invention further discloses an ear spray device dispensing a pre-defined quantity of the ear spray to the ear canal of the user. Particularly, referring to Figures 1-3, the said ear spray device includes a reservoir (115) comprising the ear protection composition, and a spray nozzle (108) having a pump mechanism.

In particular, the ear protection composition includes a combination of oils as defined in the claims.

Moreover, the spray nozzle (108) is adapted to spray the combination of oils inside the ear canal of the user when the ear spray device is in either a substantially upright or a substantially inverted position.

The pump mechanism as provided herein includes a pipette (114) enclosed in a housing (100). The said pipette (114) extends into the said reservoir (115), wherein the said ear protection composition is stored.

Further, the said pump mechanism also includes a spring (102), a secondary piston (103), a piston (104), a collar (105), a gasket (106), an actuator (110), and a closure (107) adapted to close the opening mouth of the said reservoir (115).

In various embodiments, the spring (102), the secondary piston (103), the piston (104), the collar (105), the gasket (106), the actuator (110), and a closure (107) are arranged to form the pump mechanism and are covered by the dust cap (113).

Further, a rod (112) and a rotating block (111) operatively couples the pump mechanism with the spray nozzle (108) in such a form that the said ear protection composition is applied inside the ear canal when the said ear spray is in a substantially upright or a substantially inverted position.

As such, the pump mechanism operatively couples the reservoir (115) with the spray nozzle (108) and is adapted to draw the combination of oils from the reservoir (115) spraying the combination of oils via the spray nozzle (108) inside the ear canal of the user.

As described hereinabove, the combination of oils preferably includes 99.9% w/v of *Paraffinum Liquidum* and 0.1% w/v of *Oreganum Oil,* forming a thin water repellent layer inside the ear canal of the user. The liquid coating applied to the outer ear canal specifically avoids over-pressure in the ear, and assures that bacterial formation is limited in the ear.

In accordance with one or more embodiments of the present invention, the pre-defined quantity of ear protection composition as applied topically inside the ear canal is in a metered dosage of 0.1 ml to 1.0 ml. However, other metered dosages may also be used in combination with the spray device of the present invention.

**Table 1** illustrates the preferred composition for preventing water related ear canal disorders in accordance with one or more embodiments of the present invention.

**Table 1: Composition for preventing water related ear canal disorders Technical characteristics/ parameters of the composition and device for preventing water related ear canal disorders**

| **Ingredient** | **Amount % w/v** |
|---|---|
| Paraffinum Liquidum. | 99.9 |
| Oregano Essential Oil | 0.1 |

**Table 2** illustrates general technical characteristics/parameters of the composition and ear spray device for preventing water related ear canal disorders in accordance with one or more embodiments of the present invention.

**Table 2: Technical characteristics/ parameters of the composition Discussion on Biological safety of the composition**

| **Parameter** | **Device characteristics** |
|---|---|
| Class I medical device | Yes |
| Indications for Use | For use in the Ear Canal |
| Safe composition | Yes |
| Product suitable for babies, adults and children. | Yes |
| Preservative, steroid and drug free | Yes |
| Shelf-life | 3 years |
| Sizes available | Up to 20 ml |

### 1. Biological Safety Evaluation (Safety of Liquid Paraffin)

*Paraffinum Liquidum* is a mixture of hydrocarbons having the general formula CₙHₙ and is obtained from petroleum oil before being highly refined to a very pure form using filtration and acid treatment. *Paraffinum Liquidum* is odorless and tasteless, translucent, and colorless. Paraffin is stable and should be stored below 40°C in a well closed container.

*Paraffinum Liquidum* is generally regarded as an essentially nontoxic and non-irritant material when used in topical ointments and as a coating for tablets and capsules. However, granulomatous reactions may occur following injection of paraffin into tissue for cosmetic purposes or to relieve pain. Long-term inhalation of aerosolized paraffin may lead to interstitial pulmonary disease. Ingestion of a substantial amount of white soft paraffin is known to cause intestinal obstruction.

### 2. Safety of Oregano oil

Oregano oil is known to be an irritant rather than an allergen, and a topical maximum has been set at 1.1% after which irritation can occur in some people. An adverse skin reaction study using undiluted oregano oil was severely irritating to mice, and moderately irritating to rabbits. In usual tests, the said oil tested at 2% on 25 volunteers was neither irritating nor sensitizing. Oregano oil is non-phototoxic.

In a CAM (chorioallantoic membrane of the fertile chicken egg) assay, a model for detecting irritants, Origanum Onites Oil with 57.4% carvacrol and 11.6% thymol is strongly irritating. The level of Oregano Oil incorporated in the formulation is well below that noted to cause irritation or adverse skin reactions.

### Cardiovascular effects

Origanum Vulgare oil inhibits platelet aggregation, an essential step in the blood clotting cascade.

### Reproductive toxicity

When Origanum Vulgare oil was fed to pregnant mice for two weeks at 1,000 ppm (equivalent to ~150 mg/kg), studies show there was a related increase in the rate of embryonic cell death.

### Genotoxicity

Another study investigated the use of micronucleus and comet assay to evaluate the genotoxicity of oregano oil in rats orally exposed for 90 days. The results obtained in the genotoxicity assays indicated a lack of effect in MN and standard comet assay under the conditions tested. Furthermore, no apparent oxidative damage was observed in the enzyme-modified comet assay in any of the tissues examined of rats exposed to oregano essential oil for 90 days. Therefore, this oregano essential oil appears to be safe in Wistar rats.

### Oral Toxicity

A subchronic 90-day oral toxicity study of Oregano oil was conducted in rats. Data revealed no mortality and no treatment-related adverse effects of the OEO in food/water consumption, body weight, haematology, biochemistry, necropsy, organ weight and histopathology. These findings suggest that the oral no-observed-adverse-effect level (NOAEL) of this OEO is 200 mg/kg bw in Wistar rats, the highest dose tested. In conclusion, the use of this OEO in food packaging appears to be safe based on the lack of toxicity during the sub-chronic study at doses 330-fold higher than those expected to be in contact consumers in the worst scenario of exposure.

**Table 3** illustrates the clinical properties of the composition and ear spray for preventing water borne ear canal disorders in accordance with one or more embodiments of present invention.

**Table 3: Clinical properties of composition for preventing water related ear canal disorders**

| **Clinical property** | **Ear protection composition and ear spray for preventing water borne ear canal disorders** |
|---|---|
| Intended purpose | Ear protection composition and Ear Spray as disclosed in the present invention is designed for short-term use in the ear canal and up to the ear drum to help repel and prevent water from getting trapped in the ear canal during water related activities i.e. swimming, surfing, diving etc. By repelling water from the ear, and coating the lining of the ear, the said ear protection composition and ear spray prevents extended exposure and entrapment |
| | of water in the ear, thus helping prevent conditions such as Swimmers Ear (otitis externa) and narrowing of the ear (stenosis) and associated symptoms (itching, irritation, ear redness, tenderness, swelling, pain and discharge). |
| Indications | Person who are exposed to water and who at risk of developing ear canal disorders or surfer's ear. |
| Contraindications | The ear protection composition and ear spray as disclosed in the present invention is drug free so there are no known contraindications with the product. |
| Side Effects | No known side effects as long as instructions are followed, and the product is used topically in the ear canal only. |
| | If swallowed there may be side effects and medical advice should be sought. |
| Target user groups | Users of all ages. |
| Target users | Lay persons - adults and children under supervision. Can also be used by healthcare professionals. |
| Mode of Application | For short-term use in the ear canal. |
| Duration of Use/number of reapplications | Apply 1-2 sprays to each ear before water exposure, and then repeat after 2 hours of water exposure. |
| Type of device-body interaction | Intended for short-term use in the ear canal. |

### Requirement on acceptability of side-effects

As per the evidence gathered and presented, it can be concluded that there are no known side-effects associated with the use of the ear protection composition and ear spray as disclosed in the present invention, and the said composition and spray are safe for users of all ages.

### Shelf-life

Based on the product stability studies, the ear protection composition and ear spray have been assigned a 3-year shelf-life.

### Demonstration of equivalence

The ear protection composition and ear spray are unique and have no equivalent devices available. However, the clinical, biological and technical characteristics of the product will be discussed based on the composition and properties of the ingredients used in the product.

Thus, the ear protection composition and ear spray as disclosed in the present invention have many advantages. At first, the composition is mainly cosmetic in nature. Accordingly, the composition is considered as safe and effective for preventing waterborne ear canal disorders.

The said composition does not pose any undue safety concerns to the end-users. This has been demonstrated from the study and literature reviewed on the basic ingredients as used in the said ear protection composition.

Also, no risks are associated with the use of the said composition. Moreover, no adverse events or safety issues are highlighted in any of the clinical data sources evaluated for the use of the basic ingredients as used in the ear protection composition and ear spray as disclosed in the present invention.

### Reference Signs List

- 101: Housing
- 102: Spring
- 103: Secondary Piston
- 104: Piston
- 105: Collar
- 106: Gasket
- 107: Closure
- 108: Nozzle
- 109: Dust Cap
- 110: Actuator
- 111: Rotating Block
- 112: Rod
- 113: Dust Cap
- 114: Pipette
- 115: Reservoir

## Claims

1. An ear protection composition for use in the prevention of water-related ear canal disorders, wherein the said composition is applied in an ear canal before the user enters in the water, and wherein the said composition comprises:
at least one mineral oil present in the range of 98.0 percent w/v to 99.9 percent w/v, wherein the said mineral oil is *Paraffinum Liquidum;* and at least one vegetable oil present in the range of 2.0 percent w/v to 0.1 percent w/v, wherein the said vegetable oil is an *Oreganum Oil.*

2. The ear protection composition for use as claimed in claim 1, wherein the *Paraffinum Liquidum* is in the range of 99.6 percent w/v to 99.9 percent w/v, and the *Oreganum Oil* is within the
range of 0.4 percent w/v to 0.1 percent w/v.

3. The ear protection composition for use as claimed in claim 1, wherein the disorders are due to trapping of water in an ear canal of a person.

4. The ear protection composition for use as claimed in claim 1, wherein the said *Oreganum Oil* is an essential oil selected from at least one of an *Oreganum compactum, an Oreganum vulgare,* or a combination thereof.

5. A preventing ear spray for protecting the ear from water entrapment, wherein the said spray comprises a composition for use according to any of claims 1, 3 or 4 comprising a water repellent agent, and an antimicrobial agent, wherein the said ear spray is applied in the ear canal by a metered dosage of at least 0.1 ml to 1.0 ml before the user enters in the water,
**characterized in that**
the water repellent agent is *Paraffinum Liquidum* in the range of 98.0 percent w/v to 99.9 percent w/v, and
the antimicrobial agent is an *Oreganum Oil* in the range of 2.0 percent w/v to 0.1 percent w/v.

6. The ear spray as claimed in claim 5, wherein the said *Paraffinum Liquidum* is in the range of 99.6 percent w/v to 99.9 percent w/v, and the *Oreganum Oil is* within the range of 0.4 percent w/v to 0.1 percent w/v.

7. A spray device for dispensing a pre-defined quantity of the ear protection composition
for use according to any of claims 1 to 4 in an ear canal of a user, the said device comprising:
a reservoir containing the said ear protection composition comprising a water repellent agent, and an antimicrobial agent;
a spray nozzle operatively coupled to the reservoir for spraying the said ear protection composition inside the ear canal; and
a pump mechanism connecting the said reservoir with the said spray nozzle,
wherein the pump spray mechanism is adapted to draw the said ear protection composition from the said reservoir and spray the said composition in a metered dosage via the spray nozzle in the ear canal of the user, wherein the said composition comprises
the water repellent agent *Paraffinum Liquidum,* and
the antimicrobial agent *Oreganum Oil,*
**characterized in that,**
the *Paraffinum Liquidum* is selected in the range of 99.6 percent w/v to 99.9 percent w/v, and
the *Oreganum Oil* is selected in the range of 0.4 percent w/v to 0.1 percent w/v.

8. The ear spray device as claimed in claim 7, wherein the said *Paraffinum Liquidum* is
99.9 percent w/v and the *Oreganum Oil* is 0.1 percent w/v.

9. The ear spray device as claimed in claim 7, wherein said metered dosage of the ear protection composition is 0.1 ml to 1.0 ml.

## Patentansprüche

1. Ohrenschutzzusammensetzung zur Verwendung bei der Vorbeugung von Gehörgangsstörungen im Zusammenhang mit Wasser, wobei die besagte Zusammensetzung in einen Gehörgang aufgetragen wird, bevor der Benutzer ins Wasser geht, und wobei die besagte Zusammensetzung Folgendes umfasst:
mindestens ein Mineralöl, das im Bereich von 98,0 Prozent (w/v) bis 99,9 Prozent (w/v) vorhanden ist, wobei das besagte Mineralöl *Paraffinum Liquidum* ist, und
mindestens ein Pflanzenöl, das im Bereich von 2,0 Prozent (w/v) bis 0,1 Prozent (w/v) vorhanden ist, wobei das besagte Pflanzenöl ein *Oreganum Oil* (Oreganoöl) ist.

2. Ohrenschutzzusammensetzung zur Verwendung nach Anspruch 1, wobei das *Paraffinum Liquidum*
im Bereich
von 99,6 Prozent (w/v) bis 99,9 Prozent (w/v) liegt und das *Oreganum Oil* im Bereich
von 0,4 Prozent (w/v) bis 0,1 Prozent (w/v) liegt.

3. Ohrenschutzzusammensetzung zur Verwendung nach Anspruch 1, wobei die Störungen auf das Einfangen von Wasser in einem Gehörgang einer Person zurückzuführen sind.

4. Ohrenschutzzusammensetzung zur Verwendung nach Anspruch 1, wobei das besagte *Oreganum Oil* ein ätherisches Öl ist,
das aus mindestens einem von *Oreganum compactum, einem Oreganum vulgare* oder einer Kombination davon ausgewählt wird.

5. Vorbeugendes Ohrenspray zum Schutz des Ohrs vor Wassereinfang, wobei das besagte Spray
eine Zusammensetzung zur Verwendung gemäß einem der Ansprüche 1, 3 oder 4 umfasst, die ein wasserabweisendes Mittel
und ein antimikrobielles Mittel umfasst, wobei das besagte Ohrenspray in einer abgemessene Dosierung
von mindestens 0,1 ml bis 1,0 ml in den Gehörgang eingeführt wird, bevor der Benutzer ins Wasser geht,
**dadurch gekennzeichnet, dass**
das wasserabweisende Mittel *Paraffinum Liquidum* im Bereich von 98,0 Prozent (w/v) bis 99,9 Prozent (w/v) vorhanden ist und
das antimikrobielle Mittel ein *Oreganum Oil* im Bereich von 2,0 Prozent (w/v) bis 0,1 Prozent (w/v) vorhanden ist.

6. Ohrenspray nach Anspruch 5, wobei das besagte *Paraffinum Liquidum* im Bereich
von 99,6 Prozent (w/v) bis 99,9 Prozent (w/v) liegt und das *Oreganum Oil* im Bereich
von 0,4 Prozent (w/v) bis 0,1 Prozent (w/v) liegt.

7. Sprayvorrichtung zur Abgabe einer vordefinierten Menge der
Ohrenschutzzusammensetzung zur Verwendung gemäß einem der
Ansprüche 1 bis 4 in einen Gehörgang eines Benutzers, wobei die besagte Vorrichtung Folgendes umfasst:
ein Reservoir, das die besagte Ohrenschutzzusammensetzung enthält, die ein wasserabweisendes Mittel und ein antimikrobielles Mittel umfasst;
eine Sprühdüse, die mit dem Reservoir wirkverbunden ist, um die besagte Ohrenschutzzusammensetzung in den Gehörgang zu sprühen; und
einen Pumpmechanismus, der das besagte Reservoir mit der besagten Sprühdüse verbindet, wobei der
Pump-Spray-Mechanismus dazu ausgelegt ist, die besagte Ohrenschutzzusammensetzung aus dem besagten Reservoir zu ziehen und die besagte Zusammensetzung in einer abgemessenen Dosierung über die Sprühdüse in den Gehörgang des Benutzers zu sprühen, wobei die besagte Zusammensetzung Folgendes umfasst:
das wasserabweisende Mittel *Paraffinum Liquidum*
und das antimikrobielle Mittel *Oreganum Oil,*
**dadurch gekennzeichnet, dass**
das *Paraffinum Liquidum* im Bereich von 99,6 Prozent (w/v) bis 99,9 Prozent (w/v) ausgewählt ist und
das *Oreganum Oil* im Bereich von 0,4 Prozent (w/v) bis 0,1 Prozent (w/v) ausgewählt ist.

8. Ohrensprayvorrichtung nach Anspruch 7, wobei das besagte *Paraffinum Liquidum* 99,9 Prozent (w/v) und das *Oreganum Oil* 0,1 Prozent (w/v) beträgt.

9. Ohrensprayvorrichtung nach Anspruch 7, wobei die besagte abgemessene Dosierung der Ohrenschutzzusammensetzung 0,1 ml bis 1,0 ml beträgt.

## Revendications

1. Composition pour la protection de l'oreille destinée à être utilisée dans la prévention des troubles du conduit auditif liés à l'eau, dans lequel ladite composition est appliquée dans un conduit auditif avant que l'utilisateur n'entre dans l'eau, et **caractérisée en ce que** ladite composition comprend :
au moins une huile minérale située dans la plage de 98,0 pour cent p/v à 99,9 pour cent p/v, dans lequel ladite huile minérale est du *Paraffinum Liquidum,* et
au moins une huile végétale présente située dans la plage de 2,0 pour cent p/v à 0,1 pour cent p/v, dans lequel ladite huile végétale est une *huile d'Origan* (Oreganum Oil).

2. Composition pour la protection de l'oreille destinée à être utilisée selon la
revendication 1, **caractérisée en ce que** le *Paraffinum Liquidum* se situe dans la plage
de 99,6 pour cent p/v à 99,9 pour cent p/v, et *l'huile d'Origan* se situe dans la plage
de 0,4 pour cent p/v à 0,1 pour cent p/v.

3. Composition pour la protection de l'oreille destinée à être utilisée selon la revendication 1, **caractérisée en ce que** les
troubles sont dus à l'eau coincée dans le conduit auditif d'une personne.

4. Composition pour la protection de l'oreille destinée à être utilisée selon la revendication 1, **caractérisée en ce que** ladite
*huile d'Origan* est
une huile essentielle choisie parmi au moins un *Origan compact, un Origan vulgare,* ou une combinaison de ceux-ci.

5. Spray auriculaire préventif pour protéger l'oreille et empêcher l'eau de rester coincée dedans, **caractérisé en ce que** ledit spray
comprend une composition destinée à être utilisée selon l'une des quelconques revendications 1, 3 ou 4 comprenant un agent
hydrofuge, et un agent antimicrobien, **caractérisé en ce que** ledit spray pour l'oreille est appliqué dans le conduit auditif
par un dosage calibré d'au moins 0,1 ml à 1,0 ml avant que l'utilisateur n'entre dans l'eau,
**caractérisé en ce que**
l'agent hydrofuge est du *Paraffinum Liquidum* situé dans la plage de 98,0 pour cent p/v à 99,9 pour cent p/v, et
l'agent antimicrobien est une *huile d'Origan* située dans la plage de 2,0 pour cent p/v à
0,1 pour cent p/v.

6. Spray pour l'oreille selon la revendication 5, **caractérisé en ce que** ledit *Paraffinum Liquidum*
est situé dans la plage
de 99,6 pour cent p/v à 99,9 pour cent p/v, et *l'huile d'Origan* est située dans la plage
de 0,4 pour cent p/v à 0,1 pour cent p/v.

7. Dispositif de pulvérisation pour distribuer une quantité prédéfinie de la composition
pour la protection de l'oreille destinée à être utilisée selon
l'une des quelconques revendications 1 à 4 dans le conduit auditif d'un utilisateur, ledit dispositif comprenant :
un réservoir contenant ladite composition pour la protection de l'oreille comprenant un agent hydrofuge et un agent antimicrobien ;
une buse de pulvérisation couplée de manière opérationnelle au réservoir pour pulvériser ladite composition pour la protection de l'oreille à l'intérieur du conduit auditif ; et
un mécanisme de pompe reliant ledit réservoir à ladite buse de pulvérisation, **caractérisé en ce que** le
mécanisme de pompe de pulvérisation est adapté pour déployer ladite composition de protection de l'oreille
dudit réservoir et pulvériser ladite composition en un dosage calibré via la buse de pulvérisation dans le conduit auditif de l'utilisateur, **caractérisé en ce que** ladite composition comprend
l'agent hydrofuge *Paraffinum Liquidum,* et l'agent
antimicrobien *huile d'Origan,*
**caractérisé en ce que**
le *Paraffinum Liquidum* est sélectionné dans la plage de 99,6 pour cent p/v à 99,9 pour cent p/v, et
*l'huile d'Origan* est sélectionnée dans la plage de 0,4 pour cent p/v à 0,1 pour cent p/v.

8. Dispositif de pulvérisation pour l'oreille selon la revendication 7, **caractérisé en ce que** ledit *Paraffinum Liquidum* est à 99,9 pour cent p/v et *l'huile d'Origan* est à 0,1 pour cent p/v.

9. Dispositif de pulvérisation pour l'oreille selon la revendication 7, **caractérisé en ce que** ledit dosage calibré de la composition pour la protection de l'oreille est de 0,1 ml à 1,0 ml.
